# FASCICULE DE BREVET EUROPEEN

(11) **EP 2 026 879 B1**
(45) Date de publication et mention de la délivrance du brevet: **30.03.2016**
(21) Numéro de dépôt: 07731444.1
(22) Date de dépôt: 11.05.2007
(51) Int. Cl.: A61K 8/64, A61Q 19/06

(54) **UTILISATION DE PEPTIDES EN TANT QUE PRINCIPES ACTIFS AMINCISSANTS**
VERWENDUNG VON PEPTIDEN ALS AKTIVE SCHLANKMACHENDE INHALTSSTOFFE
USE OF PEPTIDES AS ACTIVE SLIMMING INGREDIENTS

(30) Priorité: 12.05.2006 FR 0604251
(43) Date de publication de la demande: 25.02.2009
(73) Titulaire: Ashland Specialties France, 06410 Biot (FR)
(72) Inventeur: DAL FARRA, Claude, 06650 Opio (FR); DOMLOGE, Nouha, 06560 Valbonne (FR); BOTTO, Jean-Marie, 06560 Valbonne (FR)
(74) Mandataire: Macquet, Christophe
(86) Numéro de dépôt international: PCT/FR2007/000803
(87) Numéro de publication internationale: WO 2007/135268

(56) Documents cités:
- EP-A2- 1 868 632
- WO-A2-2005/002527
- FR-A1- 2 858 769
- PORCU M ET AL: "The emerging therapeutic potential of sirtuin-interacting drugs: from cell death to lifespan extension" TRENDS IN PHARMACOLOGICAL SCIENCES, ELSEVIER, HAYWARTH, GB, vol. 26, no. 2, février 2005 (2005-02), pages 94-103, XP004727629 ISSN: 0165-6147

### LISTE DES SEQUENCES

<110> Société d'Extraction des Principes Actifs
<120> Utilisation de peptides en tant que principes actifs amincissants.
<130> Bv PCT 06-62
<150> FR 0604251
   <151> 2006-05-12
<160> 6
<170> PatentIn version 3.3
<210> 1
   <211> 6
   <212> PRT
   <213> origines diverses
<400> 1
   Gly Ala Gly Ile Ser Thr
   1 5
<210> 2
   <211> 8
   <212> PRT
   <213> origines diverses
<400> 2
   Gly Ile Pro Asp Phe Arg Ser Pro
   1 5
<210> 3
   <211> 6
   <212> PRT
   <213> origines diverses
<400> 3
   Gly Ile Pro Asp Phe Arg
   1 5
<210> 4
   <211> 7
   <212> PRT
   <213> origines diverses
<400> 4
   Gly Ile Pro Asp Phe Arg Ser
   1 5
<210> 5
   <211> 7
   <212> PRT
   <213> origines diverses
<400> 5
   Gly Leu Tyr Asp Asn Leu Glu
   1 5
<210> 6
   <211> 7
   <212> PRT
   <213> origines diverses
<400> 6
   Thr Gln Asn Ile Asp Thr Leu
   1 5

## Description

L'invention concerne le domaine de la cosmétique.

La présente invention a pour objet l'utilisation de protéines de la famille des SIRT ou de fragments polypeptidiques ou peptidiques de protéines SIRT, en tant qu'agent actif amincissant, seuls ou en association avec au moins un autre agent actif, dans une composition cosmétique. Lesdits peptides sont notamment destinés au traitement de la cellulite et/ou utilisés afin de diminuer, éliminer ou prévenir les surcharges graisseuses sous-cutanées.

L'étude des gènes SIRT et des protéines correspondantes a fourni de nouvelles cibles thérapeutiques permettant d'intervenir dans la régulation du métabolisme énergétique des mammifères. Les protéines SIRT font partie de la famille des sirtuines, ce sont des protéines nucléaires, NAD+ dépendantes, responsables de la désacétylation des histones (Rine J. et Al, Genetics, 1979). Plusieurs études chez des organismes aussi divers que C. Elegans ou les mammifères ont montré qu'il existait une relation entre l'apport énergétique et la durée de vie et que la protéine SIRT était responsable de ce couplage. Ainsi une réduction de l'apport calorique augmente la durée de vie de la levure (Tissenbaum et Al, Nature*, 2001*)*.* Par ailleurs, des études récentes ont montré que SIRT1 provoque la mobilisation des graisses dans les adipocytes (Picard F. et al, Nature, 2004). Les protéines SIRT sont considérées à ce jour comme des cibles thérapeutiques potentielles pour traiter des dysfonctionnements ou des maladies liées, entre autres, à l'obésité, au diabète ou encore à l'hyperlipidémie (WO 03/061681, US 2005/0164969).

La peau est l'organe de revêtement recouvrant la totalité de la surface du corps, constituée de trois compartiments distincts superposés : l'épiderme (épithélium de revêtement), le derme (tissu conjonctif de soutien) et l'hypoderme. L'épiderme est un épithélium stratifié qui constitue la structure externe de la peau et assure sa fonction de protection. Le derme est un tissu de soutien qui participe à la résistance, à l'élasticité et surtout à la tonicité de la peau et/ou des muqueuses. Au-dessous du derme se trouve une couche de tissus adipeux : l'hypoderme.

L'hypoderme est constitué d'une couche de tissu adipeux blanc organisé en lobules rattachés à la partie inférieure du derme par des travées de fibres de collagène et de fibres élastiques. Il est constitué des grosses cellules vacuolisées, les adipocytes, presque entièrement remplies de triglycérides. Ces cellules peuvent changer rapidement de volume, lors d'un amaigrissement ou d'une prise de poids, et peuvent mesurer de 40 à 120 µm de diamètre, ce qui correspond à une variation de 27 fois en volume.

Le tissu adipeux hypodermique constitue le plus grand réservoir énergétique de l'organisme. Il est capable de stocker des lipides sous forme de triglycérides par un processus de lipogénèse puis de les libérer sous forme d'acides gras et de glycérol par un processus de lipolyse. C'est l'équilibre entre ces deux voies métaboliques qui conditionne l'adiposité. Les réserves lipidiques de l'organisme se renouvellent en permanence et sont en relation étroites avec les apports nutritionnels et les besoins énergétiques de l'organisme.

Si un déséquilibre s'installe dans l'organisme entre les processus de lipogénèse et de lipolyse, le volume et le nombre des adipocytes peut augmenter ; on parle d'hypertrophie et d'hyperplasie adipocytaire. Le développement excessif de la masse adipeuse peut alors se traduire par des modifications de l'aspect de la peau, voire évoluer vers une surcharge pondérale de l'individu pouvant aller jusqu'à l'obésité.

La cellulite, considérée comme inesthétique, correspond à une hypertrophie des cellules adipeuses par surcharge en triglycérides et une hyperviscosité de la substance fondamentale (par polymérisation des polysaccharides). Ces modifications gênent les échanges cellulaires et la mobilité des fibres conjonctives (collagène, élastine et réticuline), ce qui se traduit par une rétention d'eau, un ralentissement de la circulation veineuse et lymphatique et une perte de souplesse de la peau. L'accentuation du tissus adipeux est localisée, en particulier chez la femme, au niveau des hanches, des cuisses, des fesses, des genoux et des avants-bras. La peau prend un aspect matelassé et capitonné et au stade le plus avancé l'aspect de « peau d'orange », qui se caractérise par une succession de dépressions provoquées par un étirement excessif des travées conjonctives et l'attraction de l'épiderme vers les tissus profonds.

De nos jours, de nombreux efforts de recherche sont réalisés afin de trouver une manière efficace de lutter contre la cellulite et les surcharges graisseuses en général. De nombreuses méthodes ont été employées, comme par exemple certaines techniques médico-chirurgicales telle que la lipoplastie, le drainage lymphatique, la mésothérapie, les techniques d'ionophorèse... Cependant ces techniques, bien qu'efficaces, sont lourdes et contraignantes. Des voies biologiques ont été étudiées de façon à agir, d'une manière douce et non invasive, sur les mécanismes de formation des surcharges graisseuses sous-cutanées.

Des solutions ont été proposées pour intervenir, notamment, sur le métabolisme des acides gras. Les actifs cosmétiques et pharmaceutiques vont donc agir à différents niveaux de façon à prévenir l'apparition de cellulite. Ils vont, par exemple, favoriser la lipolyse ou bien inhiber la lipogenèse, c'est-à-dire diminuer la formation des triglycérides.

La présente invention a pour principal objectif de fournir un nouveau principe actif amincissant. En effet, les inventeurs ont réussi à sélectionner des substances particulières présentant des propriétés remarquables lorsque celles-ci sont appliquées sur la peau.

Ainsi, selon un premier aspect, la présente invention a pour objet l'utilisation d'une quantité efficace de protéines de la famille des SIRT ou de fragments peptidiques ou polypeptidiques de la famille des SIRT, seuls ou en association avec au moins un autre agent actif, pour produire un effet amincissement localisé après application sur la peau, et plus particulièrement pour réduire, éliminer ou prévenir les surcharges graisseuses sous-cutanées, les peptides appartenant à la famille des SIRT étant choisis parmi les peptides correspondants aux séquences ID N° :
(1) Gly-Ala-Gly-Ile-Ser-Thr;
(2) Gly-Ile-Pro-Asp-Phe-Arg-Ser-Pro;
(3) Gly-Ile-Pro-Asp-Phe-Arg;
(4) Gly-Ile-Pro-Asp-Phe-Arg-Ser;
(5) Gly-Leu-Tyr-Asp-Asn-Leu-Glu;
(6) Thr-Gln-Asn-Ile-Asp-Thr-Leu.

Selon une méthode de réalisation de l'invention tout particulièrement préférée, le fragment peptidique issu de la famille des SIRT possède la séquence ID N°(5), c'est-à-dire la séquence Gly-Leu-Tyr-Asp-Asn-Leu-Glu.

L'utilisation des formes variantes de ces séquences et/ou de ces fragments est décrite . Le terme « variant » désigne ici un polypeptide ou un peptide qui diffère, par exemple, de la séquence d'un peptide de référence tout en conservant ses propriétés essentielles. Généralement, les différences sont limitées de manière à ce que les séquences du peptide de référence et celles du variant soient assez similaires et, dans certaines régions, identiques.

Préférentiellement les formes variantes sont celles qui varient des séquences de référence, par la substitution d'acides aminés chimiquement équivalents (ou homologues), c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr. Le terme variant désigne ainsi un peptide qui diffère, par exemple, de la séquence d'un peptide de référence tout en conservant ses propriétés essentielles. Généralement, les différences sont limitées de manière à ce que les séquences du peptide de référence et celles du variant soient assez similaires et, dans certaines régions, identiques. Un peptide variant et un peptide de référence peuvent ainsi différer de la séquence d'acides aminés par une ou plusieurs substitutions, additions, délétions dans toutes les combinaisons.

Les fragments de protéines SIRT de nature polypeptidique ou peptidique, décrits dans la présente invention, incluent aussi l'utilisation de tous fragments biologiquement actifs, ou d'un de ses analogues ou variants. Par l'expression biologiquement actif, on entend aussi des fragments qui possèdent une activité in-vivo ou in-vitro caractéristique de l'activité du composé utilisé selon l'invention.

Dans l'invention, le terme "acide aminé" se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme "alkyl", on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées ; un polypeptide désignant un peptide de taille plus importante.

Par peptide, il faut entendre le peptide naturel ou synthétique de l'invention tel que décrit ci-dessus ou au moins l'un de ses fragments, qu'il soit obtenu par protéolyse ou de manière synthétique ou encore tout peptide naturel ou synthétique dont la séquence est totalement ou partiellement constituée par la séquence du peptide précédemment décrit.

Afin d'améliorer la résistance à la dégradation, il peut être nécessaire d'utiliser une forme protégée du peptide utilisé selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une utilisation telle que définie précédemment caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux. Les dérivés d'acides aminés et les dérivés de peptides concernent aussi les acides aminés et les peptides reliés entre eux par une liaison pseudo-peptidique. On entend par "liaison pseudo-peptidique" tous les types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents.

Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide utilisé selon l'invention peuvent être sous configuration L- et D- ; de manière préférentielle, les acides aminés sont sous forme L. Les peptides utilisés selon l'invention peuvent donc être sous forme L-, D- ou DL-.

Les peptides utilisés, décrits dans le présent brevet, peuvent être obtenus soit par synthèse chimique classique (en phase solide ou en phase homogène liquide), soit par synthèse enzymatique (Kullman et al., J. Biol. Chem. 1980, 225, 8234) à partir d'acides aminés constitutifs ou de leurs dérivés.

Les peptides utilisés selon l'invention peuvent également être obtenus par fermentation d'une souche de bactéries modifiées ou non, par génie génétique pour produire les peptides de séquence indiquée précédemment et leurs fragments, ou encore par extraction de protéines d'origine animale ou végétale, préférentiellement d'origine végétale, suivie d'une hydrolyse contrôlée qui libère les fragments peptidiques de tailles moyennes et de petites tailles, décrits dans l'invention.

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de l'hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques utilisés selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi les peptides utilisés selon l'invention peuvent être d'origine naturelle ou synthétique. Préférentiellement selon l'invention, les peptides sont obtenus par synthèse chimique.

Les peptides utilisés selon l'invention, ou la composition les contenant, possèdent une action très efficace au niveau des adipocytes. En effet, ils contribuent à diminuer significativement la quantité de triglycérides contenue dans les adipocytes de l'hypoderme.

Ce phénomène est dû vraisemblablement à un blocage du phénomène de la lipogénèse, c'est-à-dire au blocage du processus de stockage des triglycérides qui aboutit à une hypertrophie des adipocytes. Une maîtrise de la lipogénèse, c'est-à-dire de la réaction de synthèse des triglycérides dans les adipocytes, permettra d'éviter une hypertrophie des adipocytes ainsi qu'une hyperplasie consécutive. Ainsi, lorsque, en cours de différenciation adipocytaire, la quantité de triglycérides présente dans les vacuoles n'augmente pas, le volume des adipocytes et leur nombre n'augmentent pas non plus. La peau reprend progressivement son aspect «normal». Le tissu cellulitique ne se développe plus, l'effet peau d'orange est atténué. L'aspect disgracieux du corps s'estompe peu à peu.

Les peptides utilisés selon l'invention ou la composition les contenant permet ainsi, principalement en raison de leur activité particulière et prononcée sur l'adipogénèse, de prévenir l'apparition de la cellulite, de lutter contre son aggravation. Lorsque la cellulite est installée, les peptides utilisés selon l'invention ou la composition les contenant améliorent l'aspect de la peau et, par exemple, atténuent l'aspect «peau d'orange». L'ensemble de ces propriétés les rend particulièrement utiles dans des préparations cosmétiques et dermatologiques à visée amincissante.

Il est bien évident que l'invention s'adresse aux mammifères en général et plus particulièrement aux êtres humains.

Selon un deuxième aspect de l'invention, les peptides précités peuvent être utilisés dans une composition, pour le traitement topique de la cellulite et/ou de la peau d'orange. Les peptides ou la composition les contenant sont avantageusement utilisés afin de réduire, éliminer ou prévenir les surcharges graisseuses sous-cutanées.

Selon un mode de réalisation avantageux de l'invention, les peptides précités sont préalablement solubilisés dans un ou plusieurs solvants cosmétiquement acceptable, classiquement utilisé par l'homme du métier, comme l'eau, l'éthanol, le propylène glycol, le butylène glycol, le dipropylène glycol, les diglycols éthoxylés ou propoxylés, les polyols cycliques, la vaseline, le glycérol (glycérine), une huile végétale ou tout mélange de ces solvants.

Selon encore un autre mode de réalisation avantageux de l'invention, les peptides précités sont préalablement solubilisés dans un vecteur cosmétique comme les liposomes ou adsorbés sur des polymères organiques poudreux, des supports minéraux comme les talcs et bentonites, et plus généralement solubilisés dans, ou fixés sur, tout vecteur cosmétiquement acceptable.

La quantité efficace de principe actif correspond à la quantité nécessaire pour obtenir le résultat désiré.

Selon un mode de réalisation avantageux de l'invention, les peptides précités sont présents, dans les compositions de l'invention, à une concentration comprise entre 0,005 et 500 ppm (parties par million) environ, et préférentiellement à une concentration comprise entre 0,1 et 50 ppm environ en poids par rapport au poids total de la composition finale.

La composition contenant les peptides utilisés selon l'invention est une composition cosmétique, car elle est destinée à améliorer l'aspect et les performances cutanées générales de l'individu qui en fait usage.

La composition utilisée selon l'invention est préférentiellement une composition cosmétique adaptée à l'administration par voie topique cutanée comprenant un milieu cosmétiquement acceptable.

Ces compositions pourront notamment se présenter sous forme d'une solution aqueuse, hydralcoolique ou huileuse; d'une émulsion huile-dans-eau, eau-dans-huile ou émulsions multiples ; elles aussi peuvent se présenter sous forme de suspensions, ou encore poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

Ces compositions comprennent, en outre, tout additif usuellement utilisé dans le domaine d'application envisagé ainsi que les adjuvants nécessaires à leur formulation, tels que des solvants, des épaississants, des silicones, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc...

Par exemple, dans lesdites compositions, les peptides utilisés selon l'invention peuvent avantageusement être combinés avec tout autre ingrédient stimulant l'exfoliation cutanée, inhibant la lipogénèse ou stimulant la lipolyse tels que les dérivés de xanthine. On peut par exemple citer la caféine, la théophylline, la théobromine, l'acéfylline, le nicotinate de xanthinol, la diniprophylline, la diprophylline, l'étamiphylline et ses dérivés, l'étophylline, la proxyphyline, la pentophylline, la propentophylline, la pyridophylline et la bamiphylline.

Dans tous les cas, l'homme du métier veillera à ce que ces adjuvants actifs ou non actifs ainsi que leurs proportions soient choisis de telle manière à ne pas nuire aux propriétés avantageuses recherchées de la composition utilisée selon l'invention. Ces adjuvants peuvent, par exemple, correspondre de 0,01 à 20 % du poids total de la composition.

Lorsque la composition utilisée selon l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Selon un troisième aspect de l'invention, les peptides précités peuvent être utilisés dans une composition, pour le traitement topique de la cellulite et/ou des surcharges graisseuses sous-cutanées.

Selon un quatrième aspect, la présente invention concerne un procédé de soin cosmétique afin d'obtenir une action amincissante. Ainsi qu'un procédé de soin cosmétique destiné à réduire, éliminer et/ou prévenir les surcharges graisseuses sous-cutanées, et/ou destiné à lutter contre la cellulite et/ou à lutter contre le phénomène de peau d'orange, consistant à appliquer, topiquement, sur les zones concernées de la peau, une quantité efficace d'au moins un des peptides précités.

Le procédé de soin cosmétique amincissant concerne également l'application régulière de composition utilisée selon l'invention localement sur les zones du visage ou du corps à affiner, en particulier les hanches, les fesses, les cuisses, le ventre, le visage.

L'avantage de la présente invention est de pouvoir disposer d'un traitement par voie topique efficace contre l'adiposité tout en employant des méthodes « douces ».

D'autres avantages et caractéristiques de l'invention apparaîtront mieux à la lecture des exemples donnés à titre illustratif et non limitatif.

### Exemple 1 : Mise en évidence de l'activité des peptides utilisés selon l'invention sur les adipocytes.

### 1) Principe du test in-vitro.

La mise en évidence de l'activité biologique des peptides utilisés selon l'invention a été réalisée sur la lignée cellulaire préadipocytaire 3T3-L1, soumise à une différenciation en adipocytes matures. Le nombre et la taille des vacuoles lipidiques présentes dans les adipocytes matures sont évalués par observation microscopique après coloration spécifique par le colorant « Oil Red ».

### 2) Protocole expérimental.

Les cellules préadipocytaires 3T3-L1 sont ensemencées dans des Labteks et entretenues jusqu'à 100 % de confluence en milieu de culture DMEM 10 % SVF. Les cellules sont traitées avec le peptide de séquence ID n° (5), représentatif de la famille de peptides utilisés selon l'invention, mis en solution à 1 % à partir d'une solution à 50 ppm. Des contrôles non traités sont réalisés en parallèle.

Arrivées à confluence, les cellules sont cultivées en présence d'inducteurs de différenciation (IBMX, dexaméthasone et insuline), de façon à provoquer la différenciation terminale des préadipocytes en adipocytes matures. Les adipocytes différenciés sont encore incubés pendant 3 heures avec le peptide à tester à la concentration de 1%. Des contrôles non traités sont réalisés en parallèle.

Les adipocytes sont ensuite fixés durant 10 minutes dans une solution de formol 4 % et NaCl, puis la solution colorante « Oil Red» (*Sigma,* O-0625) est appliquée durant 15 minutes. Les cellules sont ensuite rincées avec de l'eau tiède et montées sur lames, en milieu hydrophile (*Aquatex*). L'observation est effectuée au microscope optique.

### 3) Résultats

Les résultats de l'observation des cellules montrent que les adipocytes matures des contrôles non traités ont une forme volumineuse sphérique et présentent une accumulation importante de vésicules lipidiques intra-cytoplasmique. Au contraire, les adipocytes matures traités par une solution contenant le peptide utilisé selon l'invention ont une morphologie moins arrondie et un contenu en vésicules lipidiques intra-adipocytaires nettement diminué.

| | Contrôle | Peptide de séquence ID n° (5) |
|---|---|---|
| Taille / nombre de gouttelettes lipidiques | ++++ | ++ |

### 4) Conclusions

La solution contenant le peptide utilisé selon l'invention s'avère particulièrement efficace dans le processus de limitation de l'hypertrophie adipocytaire. Il permet donc une élimination des triglycérides contenus dans les vésicules intra-adipocytaires.

### Exemple 2 - Préparation de compositions

### 1- Crème amincissante

| ***Noms commerciaux*** | ***Noms INCI*** | ***% massique*** |
|---|---|---|
| ***PHASE A*** | | |
| Montanov 68 | Cetearyl Alcohol (and) Cetearyl Glucoside | 5.00 |
| Squalane | Squalane | 2.50 |
| DUB IPP | Isopropyl Palmitate | 3.50 |
| Eutanol G | Octyldodecanol | 1.50 |
| Phenonip | Phenoxyethanol (and) Methylparaben (and) Ethylparaben (and) Butylparaben (and) Propylparaben (and) Isobutylparaben | 0.50 |

| ***PHASE B*** | | |
|---|---|---|
| Eau déminéralisée | Aqua (Water) | Qsp |
| Glycerine | Glycerin | 3.00 |
| Butylene Glycol | Butylene glycol | 3.00 |

| ***PHASE C*** | | |
|---|---|---|
| Simulgel EG | Sodium Acrylate/Acryloyldimethyl Taurate Copolymer(and) Isohexadecane(and) Polysorbate 80 | 0.60 |

| ***PHASE D*** | | |
|---|---|---|
| Peptide selon l'invention | | 1.25 ppm |
| Parfum | Parfum (Fragrance) | Qsp |
| Colorant | | Qsp |

### Mode opératoire

Les constituants de la phase A sont fondus à 75°C et les constituants de la phase B chauffés à 75°C. La phase A est émulsionnée à B, puis le mélange est refroidi en dessous de 40°C. Les phases C et D sont ensuite additionnées sous agitation constante.

### 2 - Spray Raffermissant - Amincissant

| ***Noms commerciaux*** | ***Noms INCI*** | ***% massique*** |
|---|---|---|
| ***PHASE A*** | | |
| Emulgade SEV | Glyceryl Stearate (and) Ceteareth-20 (and) Ceteareth-12 (and) Cetearyl Alcohol | 4.60 |
| Eumulgin B2 | Ceteareth-20 | 1.40 |
| Cetiol OE | Dicaprylyl Ether | 3.00 |
| DUB B1215 | C12-C15 Alkyl Benzoate | 5.00 |
| Phenonip | Phenoxyethanol (and) Methylparaben (and) Ethylparaben (and) Butylparaben (and) Propylparaben (and) Isobutylparaben | 0.50 |
| DUB ININ | Isononyl Isononanoate | 5.00 |

| ***PHASE B*** | | |
|---|---|---|
| Eau déminéralisée | Aqua (Water) | 15.00 |
| Glycerine | Glycerin | 3.00 |

| ***PHASE C*** | | |
|---|---|---|
| Eau déminéralisée | Aqua (Water) | Qsp |

| ***PHASE D*** | | |
|---|---|---|
| Peptide utilisé selon l'invention | | 1.50 ppm |
| Parfum | Parfum (Fragrance) | qsp |
| Colorant | | qsp |

### Mode opératoire

Les constituants de la phase A et de la phase B sont chauffés séparément à 65°C ; la phase B est incorporée à la phase A sous agitation. La température du mélange est montée à 83°C puis il est refroidi jusqu'à la température d'inversion de phase. La phase C est ensuite additionnée. L'actif est incorporé lorsque la température atteint moins de 40°C. Il est alors possible d'ajouter des parfums et/ou des colorants.

### 3 - Gel Raffermissant - Amincissant - anti-cellulite

| | ***Noms commerciaux*** | ***Noms INCI*** | ***% massique*** |
|---|---|---|---|
| 1. | Carbopol Ultrez 10 (2%) | Carbomer | 25.00 |
| 2. | Eau déminéralisée | Aqua (Water) | Qsp |
| 3. | DUB DIOL | Methyl Propanediol | 3.00 |
| 4. | EDTA | Tetrasodium EDTA | 0.10 |
| 5. | Glydant Plus Liquid | DMDM Hydantoïn (and) Iodopropynyl butylcarbamate | 0.20 |
| 6. | Peptide utilisé selon l'invention | | 1.25 ppm |
| 7. | TEA | Triethanolamine | 0.50 |
| 8. | Parfum | Parfum (Fragrance) | Qsp |
| 9. | Colorant hydrosoluble | | Qsp |

### Mode opératoire

Le gel de Carbopol est préparé à 2 %. Les ingrédients sont ajoutés dans l'ordre énuméré ci-dessus, sous agitation. Le mélange est ensuite neutralisé avec la TEA. Le parfum et les colorants sont ajoutés si nécessaire.

## Revendications

1. Utilisation d'une quantité efficace de dérivés ou fragments peptidiques de la famille des SIRT, en tant qu'agents actifs amincissants, seuls ou en association avec au moins un autre agent actif, dans une composition cosmétique, lesdits fragments peptidiques correspondants aux séquences ID N° :
(1) Gly-Ala-Gly-Ile-Ser-Thr;
(2) Gly-Ile-Pro-Asp-Phe-Arg-Ser-Pro;
(3) Gly-Ile-Pro-Asp-Phe-Arg;
(4) Gly-Ile-Pro-Asp-Phe-Arg-Ser;
(5) Gly-Leu-Tyr-Asp-Asn-Leu-Glu;
(6) Thr-Gln-Asn-Ile-Asp-Thr-Leu.

2. Utilisation selon la revendication 1 pour le traitement topique de la cellulite et/ou de la peau d'orange ; et/ou afin de réduire, éliminer ou prévenir les surcharges graisseuses sous-cutanées.

3. Utilisation selon l'une quelconque des revendications précédentes **caractérisée en ce que** la composition contient en outre un agent lipolytique cosmétiquement acceptable de préférence choisi dans le groupe des dérivés de xanthine tels que la caféine, la théophylline, la théobromine.

4. Utilisation selon l'une quelconque des revendications précédentes **caractérisée en ce qu'**au moins un groupement fonctionnel du ou des peptides est protégé par un groupement protecteur, ce groupement protecteur étant soit une acylation ou une acétylation de l'extrémité amino-terminale, soit sur une amidation ou une estérification de l'extrémité carboxy-terminale, soit les deux.

5. Utilisation selon l'une quelconque des revendications précédentes **caractérisée en ce que** les peptides sont présents dans la composition à une concentration comprise entre 0,005 et 500 ppm environ, et préférentiellement à une concentration comprise entre 0,1 et 50 ppm.

6. Utilisation selon l'une quelconque des revendications précédentes **caractérisée en ce que** les peptides sont préalablement solubilisés dans un ou plusieurs solvants cosmétiquement acceptables comme l'eau, l'éthanol, le propylène glycol, le glycérol, le butylène glycol, le dipropylène glycol, les diglycols éthoxylés ou propoxylés, les polyols cycliques, la vaseline, une huile végétale ou tout mélange de ces solvants.

7. Utilisation selon l'une quelconque des revendications précédentes **caractérisée en ce que** les peptides sont préalablement solubilisés dans un vecteur cosmétique comme les liposomes ou adsorbés sur des polymères organiques poudreux, des supports minéraux comme les talcs et bentonites, et plus généralement solubilisés dans, ou fixés sur, tout vecteur cosmétiquement acceptable.

8. Utilisation selon l'une quelconque des revendications précédentes **caractérisée en ce que** les peptides sont utilisés dans toute forme galénique adaptées à une application sur la peau, les muqueuses, les lèvres et/ou les cheveux à savoir les solutions aqueuses, hydro-alcooliques ou huileuses; les émulsions huile-dans-eau, eau-dans-huile ou émulsions multiples, les suspensions, shampoings, sticks, sprays ou encore les poudres.

9. Procédé de soin cosmétique destiné à réduire, éliminer et/ou prévenir les surcharges graisseuses sous-cutanées ; et/ou destiné à lutter contre la cellulite ; et/ou à lutter contre le phénomène de peau d'orange, **caractérisé en ce que** l'on applique sur la peau une composition contenant, comme principe actif, au moins un peptide tel que défini selon les revendications précédentes.

## Patentansprüche

1. Verwendung einer Menge von Peptidderivaten oder - fragmenten der Familie von SIRT als Wirkstoffe zum Abnehmen, allein oder in Verbindung mit mindestens einem anderen Wirkstoff, in einer kosmetischen Zusammensetzung, wobei die besagten Peptidfragmente den folgenden Sequenzen ID Nrn. entsprechen:
(1) Gly-Ala-Gly-Ile-Ser-Thr;
(2) Gly-Ile-Pro-Asp-Phe-Arg-Ser-Pro;
(3) Gly-Ile-Pro-Asp-Phe-Arg;
(4) Gly-Ile-Pro-Asp-Phe-Arg-Ser;
(5) Gly-Leu-Tyr-Asp-Asn-Leu-Glu;
(6) Thr-Gln-Asn-Ile-Asp-Thr-Leu.

2. Verwendung nach Anspruch 1 zur topischen Behandlung von Cellulitis und/oder Orangenhaut und/oder zum Verringern, Eliminieren oder Verhindern subkutaner Fettanhäufungen.

3. Verwendung nach einem der vorhergehenden Ansprüche, **dadurch gekennzeichnet, dass** die Zusammensetzung außerdem ein kosmetisch akzeptables Lipolytikum enthält, das vorzugsweise aus der Gruppe von Xanthinderivaten, wie Koffein, Theophyllin, Theobromin, ausgewählt ist.

4. Verwendung nach einem der vorhergehenden Ansprüche, **dadurch gekennzeichnet, dass** mindestens eine funktionelle Gruppe des oder der Peptide durch eine Schutzgruppe geschützt ist, wobei diese Schutzgruppe entweder eine Acylierung oder eine Acetylierung des aminoterminalen Ende oder eine Amidierung oder eine Veresterung des carboxyterminalen Endes oder beides ist.

5. Verwendung nach einem der vorhergehenden Ansprüche, **dadurch gekennzeichnet, dass** die Peptide in der Zusammensetzung in einer Konzentration, die zwischen ungefähr 0,005 und 500 ppm liegt, und vorzugsweise in einer Konzentration, die zwischen 0,1 und 50 ppm liegt, vorliegen.

6. Verwendung nach einem der vorhergehenden Ansprüche, **dadurch gekennzeichnet, dass** die Peptide vorab in einem oder mehreren kosmetisch akzeptablen Lösungsmitteln solubilisiert werden, wie Wasser, Ethanol, Propylenglykol, Glycerin, Butylenglykol, Dipropylenglykol, Ethoxy- oder Propoxydiglykole, cyclische Polyole, Vaseline, ein pflanzliches Öl oder ein beliebiges Gemisch dieser Lösungsmittel.

7. Verwendung nach einem der vorhergehenden Ansprüche, **dadurch gekennzeichnet, dass** die Peptide vorab in einem kosmetischen Vektor, wie Liposome, solubilisiert werden, oder an pulverförmigen organischen Polymeren, mineralischen Trägern, wie Talke und Bentonite, adsorbiert werden und ganz allgemein in einem beliebigen kosmetisch akzeptablen Vektor solubilisiert oder auf einem solchen fixiert werden.

8. Verwendung nach einem der vorhergehenden Ansprüche, **dadurch gekennzeichnet, dass** die Peptide in einer beliebigen galenischen Form verwendet werden, die für eine Anwendung auf die Haut, die Schleimhäute, die Lippen und/oder die Haare geeignet ist, nämlich wässrige, hydroalkoholische oder ölige Lösungen; Öl-in-Wasser-Emulsionen, Wasser-in-Öl- oder multiple Emulsionen, Suspensionen, Shampoos, Sticks, Sprays oder auch Puder.

9. Verfahren zur kosmetischen Pflege, die zum Verringern, Eliminieren und/oder Verhindern von Fettanhäufungen vorgesehen ist und/oder zum Bekämpfen von Cellulitis und/oder zum Bekämpfen des Orangenhautphänomens vorgesehen ist, **dadurch gekennzeichnet, dass** eine Zusammensetzung, die als Wirkbestandteil mindestens ein wie in den vorhergehenden Ansprüchen definiertes Peptid enthält, auf die Haut aufgebracht wird.

## Claims

1. Use of an effective quantity of peptide derivatives of the SIRT family, as slimming active agents, alone or in association with at least one further active agent, in a cosmetic composition, said peptide fragments corresponding to sequence ID Nos.:
(1) Gly-Ala-Gly-Ile-Ser-Thr;
(2) Gly-Ile-Pro-Asp-Phe-Arg-Ser-Pro;
(3) Gly-Ile-Pro-Asp-Phe-Arg;
(4) Gly-Ile-Pro-Asp-Phe-Arg-Ser;
(5) Gly-Leu-Tyr-Asp-Asn-Leu-Glu;
(6) Thr-Gln-Asn-Ile-Asp-Thr-Leu.

2. Use according to claim 1 for the topical treatment of cellulite and/or dimpling; and/or in order to reduce, eliminate or prevent excess subcutaneous fat.

3. Use according to any of the above claims **characterised in that** the composition further contains a cosmetically acceptable lipolytic agent preferably chosen from the group of xanthine derivatives such as caffeine, theophylline, theobromine.

4. Use according to any of the above claims **characterised in that** at least one functional group of the peptide(s) is protected by a protecting group, this protecting group being either an acylation or an acetylation of the amino-terminus, or on an amidation or an esterification of the carboxy-terminus, or both.

5. Use according to any of the above claims **characterised in that** the peptides are present in the composition at a concentration between 0.005 and 500 ppm approximately, and preferentially at a concentration between 0.1 and 50 ppm.

6. Use according to any of the above claims **characterised in that** the peptides are previously solubilised in one or a plurality of cosmetically acceptable solvents such as water, ethanol, propylene glycol, glycerol, butylene glycol, dipropylene glycol, ethoxylated or propxylated diglycols, cyclic polyols, petrolatum, a plant oil or any mixture of these solvents.

7. Use according to any of the above claims **characterised in that** the peptides are previously solubilised in a cosmetic carrier such as liposomes or adsorbed onto organic polymer powders, mineral substrates such as talcs and bentonites, and more generally solubilised in, or bound to, any cosmetically acceptable carrier.

8. Use according to any of the above claims **characterised in that** the peptides are used in any pharmaceutical form suitable for application on the skin, mucosa, lips and/or hair, i.e. aqueous, hydroalcoholic or oily solutions; oil-in-water, water-in-oil emulsions or multiple emulsions, suspensions, shampoos, sticks, sprays or powders.

9. Cosmetic treatment method for reducing, eliminating and/or preventing excess subcutaneous fat; and/or for combating cellulite; and/or combating dimpling, **characterised in that** a composition containing, as an active substance, at least one peptide as defined according to the above claims is applied onto the skin.
